# EUROPEAN PATENT SPECIFICATION

(11) **EP 4 385 452 B1**
(45) Date of publication and mention of the grant of the patent: **30.07.2025**
(21) Application number: 22212805.0
(22) Date of filing: 12.12.2022
(51) Int. Cl.: A61C 7/08, A61C 13/34, A61K 6/891, B29C 64/153, B33Y 10/00, B33Y 70/00, B33Y 80/00

(54) **MOLD OF BIODEGRADABLE MATERIAL FOR DENTAL ALIGNER**
FORM AUS BIOLOGISCH ABBAUBAREM MATERIAL FÜR ZAHNÄRZTLICHEN AUSRICHTER
MOULE EN MATERIAU BIODEGRADABLE POUR ALIGNEUR DENTAIRE

(43) Date of publication of application: 19.06.2024
(73) Proprietor: Stratasys, Inc., Eden Prairie, MN 55344 (US)
(72) Inventor: WITTERS, Stijn, 6167 RD Geleen (NL); DERKS, Franciscus Johannes Marie, 6167 RD, Geleen (NL)
(74) Representative: Mathys & Squire

(56) References cited:
- WO-A1-2020/163433
- CN-A- 101 804 013
- CN-A- 109 453 038
- US-A1- 2022 266 577

## Description

### Field of the invention

The invention is in the field of additive manufacturing. In particular, the invention is directed to an model of one or more teeth and/or gum, and to an additive manufacturing method for producing such a model.

### Background

Additive manufacturing processes such as stereolithography have long been employed for dental applications. In one common application, a liquid radiation curable resin composition is selectively cured via additive manufacturing processes to produce a three-dimensional mold. After production using additive manufacturing, this mold is post-cured, thermally treated, and cooled. Finally, thermoplastic sheets are shaped over the molds using thermoforming or vacuum forming to create an aligner, which is inserted into a patient's mouth for use in orthodontistry. Typically a series of molds is produced, each successive mold representing an incrementally improved realignment position of a patient's teeth.

As used herein, "aligner", or "dental aligner", also known as "clear aligner" or "invisible aligner", or "plastic orthodontic appliance," refer to orthodontic devices that are typically made of transparent plastic, that can for instance be used as a form of dental braces used to adjust teeth, or as mouth guard for preventing teeth grinding.

Aligners are described in, for example, US-B-8 019 465, assigned to Align Technology, Inc. The vacuum-forming of dental aligners over a dental aligner mold, which involves the application of considerable force and heat, is described in, for example, US-B-5 242 304, assigned to Tru-Train, Inc.

It is important that the dental aligner molds over which the dental aligners are formed possess good mechanical properties. These mechanical properties can be predicted *e.g.* by measuring the glass transition temperature (T_{g}), heat deflection temperature, and Young's Modulus.

Downsides of producing dental aligner molds using selective curing of liquid radiation curable resins is that this process can be relatively slow and/or expensive compared to other additive manufacturing processes, and that the printed parts tend to be brittle. In addition, the choice in materials that are suitable for selectively curing is limited compared to the choice of materials for other manufacturing techniques. Another downside is that articles made by selective curing of liquid radiation curable resins are typically not easily recyclable. Especially in case a series of molds is produced, in order to incrementally improve the realignment position of a patient's teeth, many molds are produced, which are used only once, and afterwards end up as unrecyclable waste.

In order for the dental aligners to be effective and comfortable, the dimensions of the dental aligner molds have to be very accurate, meaning that a 3D printing process and the used material have to be suitable for printing with high dimensional accuracy and/or surface accuracy.

In WO2020/163433A1, powder bed fusion of models for dental aligners from nylon is mentioned. However, producing dental aligner molds from nylon, *e*.*g*. polyamide 12 (PA12) also results in considerable production of unwanted waste.

Given the increasing use of plastics globally, there exists a strong and growing demand to provide more sustainable solutions. Preliminarily, additive manufacturing techniques facilitate this, as their relatively complex production method offers an enhanced degree of customization and ultimately less waste than traditional manufacturing techniques. Furthermore, several plastics are readily recyclable, which facilitates multiple functional uses from a given unit of starting material. Yet further, increasingly manufacturers are providing thermoplastic materials from bio-based, rather than petroleum-based, sources. "Bio-based" signifies that a material is synthesized in whole or part from biological origins, such as plants, animals, or micro-organisms.

It would also be desirable to use biodegradable or at least compostable plastics for the production of dental aligner molds. As is widely known, most plastics in use today, such as UV-cured resins, do not readily degrade or decompose at the conclusion of their useful life, and they are typically disposed of either by incineration or deposition in landfills. Unfortunately, many spent plastics are also increasingly entering the planet's waterways and oceans. Aside from being unsightly, this plastic build-up has a detrimental impact on certain marine life. Thus, it would be especially desirable to utilize more plastics that are "biodegradable" - that is, those which may be readily decomposed by living organisms into natural byproducts such as water, carbon dioxide, and biomass.

Many types of biodegradable plastics are known. Biodegradable plastics may originate from bio-based or petroleum-based sources. From a sustainability standpoint, bio-based plastics are typically preferred.

WO2019/043137A1, assigned to Evonik Rohm Gmbh, is directed to biocompatible polymeric powders (with examples based on polylactic acid (PLA, PLLA) and polycaprolactone (PCL)) to be used for 3D printing applications.

However, articles made from such biodegradable and/or biobased powders typically have unfavorable mechanical properties, and are often not very heat resistant, making them unsuitable for use as a dental aligner mold, because of the heat involved in thermoforming dental aligners over a dental aligner mold.

Despite the foregoing attempts, an unmet need exists to provide dental aligner molds that are biodegradable (or compostable), while still offering the desired dimensional accuracy, surface accuracy, and/or dimensional stability.

An object of the invention is to provide a material and/or a method that can be used to provide biodegradable and/or compostable dental aligner molds.

### Summary of the invention

The inventors have surprisingly found that this object can be met, at least in part, by producing dental aligner molds from polyhydroxyalkanoate (PHA) material.

Therefore, according to a first aspect of the invention, there is provided a positive model of one or more teeth and/or gum according to claim 1, suitable for producing a dental aligner, the model comprising a polyhydroxyalkanoate (PHA).

According to a second aspect of the invention, there is provided an additive manufacturing method according to claim 7 for producing a such a model, comprising sintering a powder comprising a polyhydroxyalkanoate (PHA).

The inventors have found that articles with high dimensional accuracy and dimensional stability can be produced by powder bed fusion of a powder comprising polyhydroxyalkanoate (PHA). Surprisingly, even despite the fact that PHA materials have a lower melting temperature and are not as strong as PA 12, which is a material that is typically used for powder bed fusion, the model according to the invention has enough dimensional accuracy for being used as a dental aligner mold. In addition, the model can withstand the conditions for thermoforming of dental aligners, even though the temperatures used in thermoforming are very close or in some cases even higher than the melting temperature of the PHA material. Under the same conditions, other biodegradable materials such as polylactic acid (PLA), despite having a higher melting point than certain suitable PHA materials, were found to be not suitable for a dental aligner mold.

### Brief description of the drawings

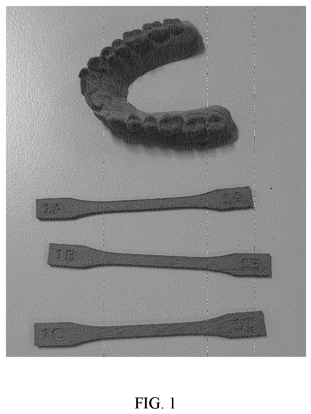

Figure 1 shows a photograph of a dental aligner mold and test articles produced from PHBx.

### Detailed description of the invention

According to a first aspect of the invention, there is provided a positive model of one or more teeth and/or gum, suitable for producing a dental aligner, the model comprising a polyhydroxyalkanoate (PHA).

Although PHA polymers encompass a greater number of species than the PHBx polymers elsewhere described herein, *infra,* a preferred subset of PHAs includes those in the PHBx group. Particularly preferred amongst PHAs useful as a powder include poly-3-hydroxybutyrate (P3HB), poly-4-hydroxybutyrate (P4HB), polyhydroxyvalerate (PHV), polyhydroxyhexanoate (PHH), polyhydroxyoctanoate (PHO), polyhydroxydecanoate (PHD), polyhydroxydodecanoate (PHDD), or copolymers thereof. Accordingly, in an embodiment, the PHA powder comprises, consists of, or consists essentially of P3HB, P4HB, PHV, PHH, PHO, PHD, PHDD, or any combination thereof. In a particularly preferred embodiment, the PHA powder comprises, consists of, or consists essentially of P3HB.

In preferred embodiments, the PHA comprises, consists essentially of, or consists of a homopolymer or copolymer of 3-hydroxy butyric acid. 3-hydroxy butyric acid, also known as β-Hydroxybutyric acid, is an organic compound and a beta hydroxy acid with the chemical formula CH3CH(OH)CH2CO2H; its conjugate base is β-hydroxybutyrate, also known as 3-hydroxybutyrate. β-Hydroxybutyric acid is a chiral compound with two enantiomers: D-β-hydroxybutyric acid and L-β-hydroxybutyric acid. Its oxidized and polymeric derivatives occur widely in nature. 3-hydroxy butyric acid is a precursor to poly(3-hydroxybutyrate) (P3HB), which is a biodegradable polyester. The chemical structure of P3HB is depicted below:

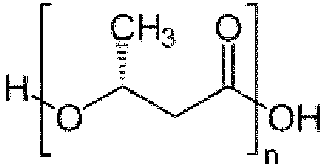

In embodiments, the powder composition comprises a homopolymer or copolymer of 3-hydroxy butyric acid. In various embodiments, the powder composition comprises a (co)polymer of 3-hydroxy butyric acid and an additional acid. The additional acid may be of any suitable type which assures compatibility and biodegradability of the resultant polymer, but particularly preferred additional acids include 3-hydroxy hexanoic acid, 3-hydroxy valeric acid, and 4-hydroxy butyric acid. As used herein, copolymers of 3-hydroxy butyric acid and 3-hydroxy hexanoic acid are referred to as "PHBH", whereas copolymers of 3-hydroxy butyric acid and 3-hydroxy valeric acid are referred to as "PHBV". Copolymers of 3-hydroxy butyric acid and 4-hydroxy butyric acid are referred to as "P3HB4HB". Furthermore, as used herein, the general nomenclature for a homopolymer of 3-hydroxy butyric acid, or a copolymer of 3-hydroxy butyric acid with any additional acid(s) is referred to herein under the general nomenclature "PHBx".

If used, the comonomers may be incorporated into the 3-hydroxy butyric acid in any quantity relative to each other as is desired, such as in an amount from 1 to 50 mol%, preferably 1 to 15 mol%, more preferably 5 to 12 mol%.

In order to withstand the conditions for thermoforming dental aligner or other orthodontic devices, the PHA of the model has melting peak temperature of 135 °C or higher, preferably 140 °C or higher. The melting temperature of the PHA may even be as high as 190 °C, such as 140-180 °C, or 145-175 °C.

As used herein, melt behavior of materials, such as melting peak temperature, melting onset temperature, and crystallization onset temperature are measured according to ISO 11357-1/3. Unless otherwise indicated, the terms melting point and melting temperature refer to the melting peak temperature of a material.

In embodiments, the melting peak temperature of the PHA of the model is higher than the temperature used for the thermoforming process used to make dental aligner. Surprisingly, the model can even be suitable for being used as a dental aligner mold when the melting peak temperature of the PHA is close to or lower than the temperature used for the thermoforming process. This can at least partly be explained by the fact that dental aligner molds are typically subjected to the thermoforming conditions only briefly, for instance for a duration of 30 seconds - 2 minutes.

Other biodegradable materials, notably polylactic acid (PLA), do not show similar stability as PHA at temperatures close to their melting peak temperature. In order to predict the stability of a material at elevated temperatures, parameters or measurements such as heat deflection temperature (HDT) and/or dynamic mechanical thermal analysis (DMTA) may be applied. Apart from the preferred PHA materials described above, the skilled person may select other suitable PHA materials for the models based on these parameters and the conditions used for thermoforming of dental aligners.

In an embodiment, the model may further comprise one or more additive components. Such additives may be any suitable additive, for instance used for 3D printing, without limitation, such as flame retardants, flow aids, fillers, pigments, and stabilizers. Suitable flow aids include fumed silicas, precipitated silicas; suitable fillers include glass particles and glass fibers (having a length of no more than 100 microns, but preferably less), glass beads, metal particles, and ceramic particles, suitable pigments include titanium dioxide, particles based on rutile, particles based on anatase, carbon black particles, carbon fibers; and stabilizers such as heat stabilizers and UV-stabilizers. If used, in an embodiment, one or more flow aids may be included in an amount from 0.05 to 20 wt.%, relative to the entire model.

A variety of additional additives are known that can be incorporated into models according to the present invention. Additives that may be present in the mold include additives for additive manufacturing which include, for example, flow aids (other than the monomeric, oligomeric, or polymeric flow aids described elsewhere herein), fillers (including dispersed reinforcing materials such as chopped or milled glass fibers, chopped or milled carbon fibers, nano-fillers, clays, wollastonite and micas, as well as continuous reinforcing materials), pigments, processing aids (such as mold release agents), leveling agents, degassing agents, stabilizers (such as antioxidants and UV stabilizers), plasticizers, impact modifiers (core/shell rubbers), and carrier polymers.

Other examples of fillers which are known and commonly used in thermoplastic resin compositions include mineral fillers such as clay, mica, talc, and glass spheres or beads. Reinforcing fibers are for example glass fibers. An advantage of a resin composition comprising glass fibers is its increased strength and stiffness, particularly also at higher temperatures, which allows use at temperatures up to close to the melting point of the polymer in the associated composition.

Inorganic substances are especially suitable as fillers because of their tendency to impart water-resistance, heat-resistance, and robust mechanical properties into the composition. In an embodiment of the invention, the filler is inorganic and comprises ceramics such as silica (SiO2) nanoparticles, i.e., those particles having a mean particle size of from between 1 nanometer (nm) to 999 nm, or between 5 nm - 800 nm; or microparticles, i.e., those particles having a mean particle size of between 1 micrometer (µm) to 999 µm, or from 1 - 100 µm . Average particle size may be measured using a variety of techniques, such as scanning electron microscopy or laser diffraction particle size analysis in accordance with ISO13320-1. Please see US 6,013,714 for further examples of silica nanoparticles.

In other embodiments of the invention, alternative inorganic filler substances may be present, such as those containing glass or metal particles. Certain non-limiting examples of such substances include: glass powder, alumina, alumina hydrate, magnesium oxide, magnesium hydroxide, barium sulfate, calcium sulfate, calcium carbonate, magnesium carbonate, silicate mineral, diatomaceous earth, silica sand, silica powder, oxidation titanium, aluminum powder, bronze, zinc powder, copper powder, lead powder, gold powder, silver dust, glass fiber, cellulose fibers, cellulose nanocrystals, titanic acid potassium whiskers, carbon whiskers, sapphire whiskers, verification rear whiskers, boron carbide whiskers, silicon carbide whiskers, and silicon nitride whiskers.

In an embodiment, however, a model according to the present invention is substantially devoid of any fillers at all. The absence of fillers may be beneficial because it ensures improved workability (i.e. flowability, surface finish) of the model.

Suitable impact modifiers are rubber-like polymers that not only contain apolar monomers such as olefins, but also polar or reactive monomers such as, among others, acrylates and epoxide, acid or anhydride containing monomers. Examples include a copolymer of ethylene with (meth)acrylic acid or an ethylene/propylene copolymer functionalized with anhydride groups. The advantage of impact modifiers is that they do not only improve the impact strength of the resin composition but also contribute to an increase in viscosity. A suitable impact modifier is, for example, a maleic anhydride functionalized polyolefin.

Colorants, such as pigments or dyes, may optionally also be present in various embodiments. As colorants, for example, carbon black or nigrosine can be employed. EP2935430 describes various other common pigments, which may be used suitably herein, including titanium dioxide in one or more of its three crystalline forms (rutile, anatase, and brookite), ultramarine blue, iron oxides, bismuth vanadates, effect pigments including metallic pigments such as aluminum flake and pearlescent pigments such as micas, and organic pigments, for example phthalocyanines, perylenes, azo compounds, isoindolines, quinophthalones, diketopyrrolopyrroles, quinacridones, dioxazines, and indanthrones.

The model may additionally include one or more stabilizers. The presence of stabilizers is optional. Stabilizers are known per se and are intended to counter deterioration as a result of the effects of for example heat, light and radicals thereby formed. Known stabilizers that can be present in the model are for example hindered amine stabilizers, hindered phenols, phenolic antioxidants, copper salts and halogenides, preferably bromides and iodides, and mixtures of copper salts and halogenides, for example copper iodide/potassium iodide compositions and also phosphites, phosphonites, thioethers, substituted reorcinols, salicylates, benzotriazoles, hindered benzoates, and benzophenones. Preferably, the stabilizer is chosen from the group consisting of inorganic, hindered phenolic oxidant, hindered amine stabilizer and combinations thereof. More preferably, the stabilizers are a combination of inorganic stabilizer, a phenolic antioxidant and a hindered amine. In an embodiment, if the model includes a stabilizer constituent, such constituent is present by weight, relative to the entire composition, from about 0.05 wt.% to about 2.0 wt.%, or from about 0.1 to 1.5 wt.%, or from 0.3 wt.% to 1.2 wt.%.

In an embodiment, the model also includes one or more lubricants. Such substances include long chain fatty acids, especially stearic acid or behenic acid, salts thereof, especially Ca or Zn stearate, as well as their ester derivatives or amide derivatives, in particular ethylene-bis-stearylamid, montan waxes and low molecular weight polyethylene or polypropylene waxes. In an embodiment, suitable lubricants include esters or amides of saturated or unsaturated aliphatic carboxylic acids having 8 to 40 carbon atoms with saturated aliphatic alcohols or amines having from 2 to 40 carbon atoms, and metal salts of saturated or unsaturated aliphatic carboxylic acids with 8 to 40 carbon atoms used with ethylene bis-stearyl amide, and calcium stearate.

The aforementioned lists of additives are not intended to be limiting, and any other suitable additive may be employed as is generally known to those of skill in the art to which this invention applies. Further such examples include UV stabilizers, gamma ray stabilizers, hydrolysis stabilizers, thermal stabilizers, antistatic agents, emulsifiers, nucleating agents, drip agents (such as polytetrafluoroethylene or polyvinylpyrrolidone), and plasticizers.

If included, the additives described herein may be used singly or in combinations of two or more. If additives are present at all, the model can comprise from 0.001 wt.% to 20 wt.%, or from 0.1 wt.% to 20 wt.%, or from 0.5 wt.% to 20 wt.% of additives, relative to the total weight of the model.

In view of biodegradability of the model powder composition and articles made thereof, it may be preferred that the amount of pigments and/or other additives is low compared to the amount of PHA. Therefore, the amount of PHA relative to the total weight of the model is 80 % or more, more preferably 90% or more, even more preferably 95 wt.% or more. The amount of PHA may even be as high as 99.95 wt.%. For instance, the amount of PHA in the model may be 92-99 wt.% or 94-97 wt.%. The amount by weight of the PHA, relative to the total weight of the entire model, is 80-99.9 %.

Preferably, for instance in order to withstand the heat applied when thermoforming a dental aligner around a dental aligner mold, the model has a heat deflection temperature at 1.8 MPa of 70 °C or higher, preferably 100 °C or higher, more preferably 120 °C or higher , such as 80-175 °C, measured according to ASTM D648.

Alternatively or additionally, the model preferably has a tensile modulus as measured according to ASTM D638 of 800 MPa or higher, preferably 1500 MPa or higher, more preferably 2000 MPa or higher, such as 1200-3000 MPa.

Alternatively or additionally; the model preferably has an elongation at break as measured according to ASTM D638 of 2 % or higher, more preferably 4% or higher, even more preferably 5 % or higher, such as 3-20 %.

Alternatively or additionally; the model preferably has an ultimate tensile strength of 10 MPa or higher according to ISO 527-1/2, more preferably 15 MPa or higher, even more preferably 20 MPa or higher. The ultimate tensile strength may be as high as 150 MPa or even higher. For instance, the ultimate tensile strength may be 10-50 MPa.

According to a second aspect of the invention there is provided an additive manufacturing method for producing a model of one or more teeth and/or gum as described herein, comprising sintering a powder comprising a polyhydroxyalkanoate (PHA).

The additive manufacturing method as described herein may comprise the steps of: (a) providing a powder having a D50 particle size value of 20-150 µm, comprising a polyhydroxyalkanoate (PHA), the powder having a crystallization onset temperature T_{c,onset} and a melting peak temperature T_{m,peak} measured according to ISO 11357-1/3, (b) subjecting the powder to powder bed fusion, wherein the temperature of the powder bed is between the crystallization onset temperature and the melting peak temperature, thereby forming a physical model of the one or more teeth and/or gum.

Suitable powder bed fusion techniques include Selective Laser Sintering (SLS), High Speed Sintering (HSS), Selective Absorption Fusion (SAF), or MultiJet Fusion (MJF). Preferably, SLS and/or SAF are used.

Preferably, the PHA is a homopolymer or copolymer of 3-hydroxy butyric acid, preferably a copolymer of 3-hydroxy butyric acid, with 3-hydroxy hexanoic acid and/or 3-hydroxy valeric acid as comonomer.

In case SLS is used as powder bed fusion technique, a high density, high energy source of radiation, such as a laser, is used to selectively melt or fuse a portion of particles into a desired shape. A control mechanism serves to direct both the path and intensity of the laser in order to fuse powder disposed within specified boundaries, often on a layerwise basis. Each layer, or "slice," represents a cross-section of the final component to be fabricated at a specified thickness. Machine controls operate selectively to sinter sequential layers of powder, producing a completed part comprising a plurality of slices sintered together. Preferably, the machine control mechanism is computer-directed, and utilizes CAD files of varying formats to determine the defined boundaries for each slice.

The part may be produced by depositing a first portion of sinterable powder onto a target surface of a part bed, scanning the directed laser over the target surface, and sintering a first layer of the first portion of powder on the target surface to form the first slice. The powder is thus sintered by operating the directed laser beam within the boundaries defining the first slice, with sufficient energy, or fluence, to sinter the powder. The first slice corresponds to a first cross-sectional region of the part.

A second portion of powder may then be deposited onto the surface of the part bed and that of the first sintered slice lying thereon, and the directed laser beam scanned over the powder overlying the first sintered slice. A second layer of the second portion of powder is thus sintered by operating the laser beam within the boundaries which then define the second slice. The second sintered slice is formed at a temperature sufficient to sinter it to the first slice, with the two slices fusing together into a single portion of the object to be built. Successive layers of powder are deposited onto the previously sintered slices, with each layer being sintered in turn to form an additional slice.

Different types of directed lasers can be used as high density, high energy source of radiation to selectively melt or fuse a portion of particles into a desired shape. In order to melt or fuse a portion of particles, the particles should absorb at least some of the light that is emitted by the lasers. If the particles do not absorb enough of the laser's light, pigments or dyes can be added to increase light absorption. Gas lasers, such as CO₂ lasers, are often used in powder bed fusion processes, because of their high power and efficiency. CO₂ lasers typically emit light with a wavelength of 9.6-10.6 microns, and can have a power output of up to 100 W or even higher. Polyamide and polyester materials that are typically used for powder bed fusion processes, including bio-based and/or biodegradable materials absorb a portion of the light emitted by CO₂ lasers. However, CO₂ lasers are typically large, complex and expensive. Also, CO₂ lasers are very sensitive for proper alignment. This makes CO₂ lasers less suitable for *e*.*g*. small companies and home users of that want to use powder bed fusion processes. Therefore, smaller powder bed fusion printers with other types of lasers have recently gained attention. For instance, laser diodes with a wavelength in the near-infrared (NIR) region, *e*.*g*. around 800 nm, are also used for powder bed fusion. Common wavelengths for such diode lasers are 808 nm, 976 nm, or 1065 nm. Such laser diodes typically have a much lower power output than CO₂ lasers, for instance around 5-30 W. Polyamide and polyester materials typically do not absorb enough light in the NIR region. Therefore, in order to be suitable for powder bed fusion using a NIR laser, the particles are often mixed with a pigment or a dye that absorbs light in the NIR region. However, addition of too much pigment can negatively influence other properties of the powder, such as the mechanical properties of the printed part. In addition, especially when a biodegradable part is made, addition of too much pigment can negatively influence biodegradability.

In view of the above, and depending of the type of laser that may be used for sintering, the powder may further comprise a pigment and/or other additives, for instance in the amounts described above for the model. In embodiments, the powder comprises a pigment, wherein the weight of pigment relative to the total weight of the powder is 2.0 % or less by weight or less relative to the total weight of the powder, preferably 1.5 wt.% or less, more preferably 1.0 wt.% or less, even more preferably 0.5 wt.% or less, such as 0.4 wt.% or less, or even 0.3 wt.% or less. The amount of pigment may even be as low as 0.05 wt.%, but will typically be higher.

Preferably, especially when a low power laser diode is used, the pigment absorbs light in the near-infrared region of the electromagnetic spectrum. This means that the powder composition can be heated effectively by irradiation with a NIR laser. Preferably, the pigment has a reflectivity at 800 nm of 20 % or less, preferably 10 % or less. Preferably, the pigment is biodegradable, compostable, or does not harm the environment when the powder composition or an article is degraded.

Preferably, the powder has a reflectance at a wavelength of 800 nm of 50 % or less, preferably 30 % or less, more preferably 25 % or less.

For using the model as a dental aligner mold, dimensional accuracy may be an important factor. Therefore, the additive manufacturing method may further comprise providing a digital 3D model of one or more teeth and/or gum according to which the physical model is made, and the parameters are preferably chosen such that the 80% quantile of the model formed in step (b) compared to the digital 3D model is 0.5 mm or less, preferably 0.3 mm or less, more preferably 0.2 mm or less. The exact parameters of the equipment that is used to carry out the powder bed fusion step of the additive manufacturing method, typically a 3D printer, are dependent on the specific combination of powder and equipment used.

The temperature of the powder bed during powder bed fusion is preferably 50 °C or less below the melting peak temperature of the powder composition, more preferably 40 °C or less below the melting peak temperature of the powder composition, such as 30 - 5 °C, or 25 - 10 °C below the melting peak temperature of the powder composition.

The melting peak temperature of the PHA depends on the type of monomer and comonomer used. If for instance PHBV powder comprising ca. 2 wt.% valerate having a melting peak temperature of 175 °C is used, the temperature of the powder bed during additive manufacturing may for instance be 140-170 °C. If for instance PHBH powder comprising ca. 6 wt.% of hexanoate having a melting peak temperature of 145 °C is used, the temperature of the powder bed during additive manufacturing may for instance be 110-140 °C.

In embodiments, in order to obtain a powder that is especially suitable for powder bed fusion, the method further comprises one or more steps selected from: providing a particulate starting material comprising a homopolymer or copolymer of 3-hydroxy butyric acid; compacting the starting material with a compacting pressure of > 5 kN/cm², thereby obtaining a compacted material; reducing the size of the particulate starting material or the compacted material; heating the starting material, the compacted material and/or the size-reduced material to a temperature sufficient to prevent sticking of the starting material or compacted material, thereby obtaining an annealed material; mixing the starting material, the compacted material, the size-reduced material or the annealed material, with a pigment, preferably using high shear mixing; size fractionating the starting material, the compacted material, the size-reduced material, the annealed material, and/or the mixed material.

In embodiments, the starting material, consists of, or consists essentially of a P3HB polymer. At standard temperature and pressure, the P3HB polymer may take any suitable form, such as a powder, flake, or granule. Of these, powders are preferred.

In an embodiment, the method according to the first aspect of the invention involves the optional step of reducing the size of the starting material to form an size reduced material with, *i.a.,* a desired average particle size or particle size distribution. This step is necessary if the starting material is in the form of flakes, granules, or another powder with an average particle size that is larger than the desired end average particle size of the ultimate build material for the specified additive manufacturing process.

The size reduction may include a milling or micronizing process to provide a particle size suitable for the intended printing process. Milling can be done at or around room temperature (*e*.*g*. 10 to 30 °C) but may be lower for other methods such as cryogenic milling. With cryogenic milling, or cryo-milling, the polymer is cooled down with liquid nitrogen (alternatives to N₂ include solid or liquid carbon dioxide) to prevent softening and clogging of the apparatus during milling.

Other well-known milling techniques include jet milling and mechanical milling. Jet milling processes, for example, grind materials by using a high-speed jet of compressed air or inert gas to impact particles into each other. Jet mills can be designed or used to output particles below a certain size, while continuously milling particles above that size, resulting in a narrow size distribution of the resulting product. Particles leaving the mill can be separated from the gas stream by cyclonic separation.

Milling techniques, especially mechanical milling, may be carried out in a pinned-disk mill, a fluidized-bed opposed jet mill, or a baffle-plate impact mill. Regardless of the milling technique and equipment used (all of which is well-known in the art to which this invention applies), the process should be carried out such that the resulting particle size distribution has a median particle size D50 is in the range from 1 to 650 microns, or more preferably from 1 to 400 microns, or for example, 10 to 200 microns, 20 to 100 microns, or 40 to 50 microns. Median particle size D50 is determined via laser diffraction particle size analysis in accordance with ISO 13320-1. In particular, particle size analysis using a SYMPATEC HELOS/H3982 can be used to determine the particle size distribution.

In a preferred embodiment, the starting material or the size reduced starting material has a particle size distribution with a D50 particle size in the range of 20 to 100 microns, or 30 to 90 microns, or 30 to 80 microns, or 40 to 90 microns, or 40 to 80 microns, or from 40 to 50 microns. A narrow particle size distribution having a mean particle at the sizes listed is desirable because it tends to improve the flowability of the ultimate powder created therefrom. This ensures superior processing and a reduction in agglomeration when such powder is used in powder-based additive manufacturing processes, such as multi-jet fusion or selective laser sintering.

In a preferred embodiment, the size reduction step comprises a jet milling or mechanical grinding process, wherein the jet milling or mechanical grinding process is carried out at a temperature of 15 to 35 °C, or from 15 to 30 °C.

If the particle size distribution of the starting material, the size reduced material or mixed material is too broad, or if a narrower particle size distribution is desired, it is possible to perform a size fractionation step in order to obtain a narrower particle size distribution. Size fractionation may be conducted one or more times so as to select a subset of the particles, flakes, or granules contained in the material for further operation or use. This is typically achieved via sifting or sieving, and the apparatus for sifting and/or sieving may be selected with one or more screens or partitions so as to filter out particles above and/or below any specified size. In an embodiment, the starting material is sieved to assure that particles below 30 microns and above 100 microns are not maintained to any substantial degree. Size fractionation may be performed at any point or points along the process, but it is commonly carried out on the starting material or after the size reduction step, or it is not performed at all. In an alternative embodiment, the size fractionation step is performed prior to or after more than one step in the process, or even prior to or after each step in the process. Preferably, size fractionation is performed as a final step to assure the obtained powder composition is optimized for suitability as a powdered build material for use in additive manufacturing processes.

The starting material or the size-reduced material may be mixed with a pigment, for instance using high shear mixing. High shear mixing refers to any mixing in which the solid material is subjected to substantial amounts of shear. This can be achieved using a wide range of different type of mixers. Suitable mixers for high shear mixing include conical screw mixers, also known as Nauta^{®} mixers; conical paddle mixers; and cyclomixers. A suitable way to determine the amount of shear provided by mixers with a rotating element, such as paddle mixers, blade mixers or screw mixers is the tip speed of the rotating element. Preferably, high shear mixing comprises mixing using a rotating element having a tip speed of at least 1.0 m/s, more preferably 3.0 m/s, even more preferably 5.0 m/s or more. The tip speed may be as high as 200 m/s, but will typically be lower than that, for instance 50 m/s or less, such as 10-40 m/s, or 15-30 m/s. Apart from mixers with a rotating element, other types of equipment, for instance shakers, may also be used for high shear mixing. In order to determine whether mixing using a different type of mixer than a mixer with a rotating element qualifies as high shear mixing in the context of this invention, the performance of such a non-rotating mixer can be compared to a mixer with a rotating element (e.g., a conical paddle mixer from Hosogawa Micron) by comparing the NIR reflectance over time when mixing the starting material or size reduced material with a pigment.

The high shear mixing can comprise one or more mixing steps. For instance, the high shear mixing can comprise a step of mixing the starting material or the size reduced material with the pigment in a low-shear mixer (*e*.*g*. a Lödige mixer), followed by a step of shaking using high shear.

Using high shear mixing, it is possible to produce a powder composition with lower NIR reflectance compared to low shear mixing when using the same materials, and/or the minimum NIR reflectance can be achieved in a shorter amount of time. Long mixing times are unwanted, because of economic reasons and because they slow down production of the powder composition. Therefore, preferably, the high shear mixing step is performed for 90 minutes or less, more preferably 60 minutes or less, even more preferably 30 minutes or less. For instance, the high shear mixing step is performed for 0.5-15 minutes, or 1-10 minutes.

In some embodiments according to the first aspect, the starting material is first subjected to a compacting step in order to increase the free bulk density (FBD) of the starting material. In a preferred embodiment, the compacting is carried out by any suitable apparatus for compacting such materials, including, for example a Bepex Labor Kompaktor. The force and/or pressure applied to the starting material may vary as desired, but in a preferred embodiment, the compacting is carried out such that it imparts a pressure of greater than 5 kN/cm², more preferably greater than 25 kN/cm², more preferably greater than 40 kN/cm², or from 5 - 400 kN/cm², or from 25 - 300 kN/cm², or from 40 - 200 kN/cm² on the material. Inventors have surprisingly discovered that the beneficial effects of this compacting step remain with the starting material even after the subsequent downstream processes described elsewhere herein such that the resulting processed material possesses enhanced suitability for use in additive manufacturing applications. If a compacting step is performed, it is preferably performed prior to size reduction and/or mixing of the starting material or size reduced material with a pigment.

For instance, if the starting material comprises low-density PHBx, compacting the starting material may be performed in order to obtain PHBx with a density of 0.4 g/mL or higher, preferably 0.5 g/mL or higher, such as 0.5-0.8 g/mL.

According to some embodiments of the first aspect, the starting material may be heated to produce an annealed material. Preferably, such a heating step heats the material to a temperature sufficient to prevent sticking of the compacted material. The heating process preferably involves annealing in the sense that the material is heated up to some temperature below its glass transition temperature or peak melting temperature for a moderate period of time, after which it is allowed to cool again in order to reduce internal stresses therein, and in order to increase the melting onset temperature. Preferably, if performed, the heating step is performed before the mixing step. If a compacting step is also performed, the heating step is preferably performed after the compacting step. The heating step can be performed before a size reduction step (*e.g.* on granules) and/or after the size reduction step, *i.e.,* on the powder.

The method of heating will differ according to several factors as will be appreciated by the skilled artisan to which this invention relates, including but not limited to the desired quantity of material to be heat-treated. Overarching common techniques involve batch and continuous heating. Batch heating typically involves forced convection in an oven, although for lab-scale quantities, other methods may be employed, such as the insertion of a flask containing the material into a hot oil bath under vacuum while purging with an inert gas or a tumble dryer. Continuous heating, meanwhile, typically involves passing the material along a conveyor in a heated tube or chamber. This method allows for less restricted air flow and is more suitable for industrial scale heating processes. Whether batch or continuous heating processes are utilized, other heating methods, such via the application of infrared radiation, may be employed as a substitute for the aforementioned convection heating.

Regardless of the general technique, the process will typically involve heating the material to a (i) desired temperature at a (ii) controlled rate, maintaining this desired temperature for (iii) a specified period of time, and then cooling the material at a (iv) controlled rate to a (v) final temperature. Any of (i) - (v) can vary as desired as will be appreciated by the skilled artisan, it being understood further that different PHBx polymers will necessitate varying conditions as they will possess intrinsically different material characteristics.

In various embodiments, however, (i) is selected as being up to 120 °C, or 130 °C, or 135 °C, or 140°C, or 150 °C, or 160°C, or 170 °C. The ultimate desired temperature (i) should be selected so as not to exceed the peak melting temperature for the material being utilized, although it may at least briefly exceed material's melting point onset temperature (T_{m,onset}). In other embodiments, (i) may vary depending on the material, such as from 100 to 200 °C, or from 120 to 180 °C, or from 130 to 160 °C.

Despite the ultimate heating temperature, a variety of (ii) heating rates may be selected, such as from 1 °C / h to 100 °C / h, or from 2 °C / h to 25 °C / h, more preferably 5 °C / h to 10 °C / h, although other rates may be employed as suitable. The rate of (ii) heating is not necessarily as important as (i) or (iii), provided that the material is heated evenly and does not exceed the peak melting temperature locally. Of course, it will be appreciated that a rapid heating is desired to minimize the overall processing time.

Furthermore, it is appreciated that the heating may occur continuously or in a stepwise processes; that is, the material may be heated to a certain intermediate value below the ultimate desired peak temperature (i) , maintained there for a specified period of time, then ramped up further again as appropriate. This stepwise process may occur in the presence of two or more than two so-called intermediate desired temperatures with one, two, or more than two intermediate maintenance times in between.

Once the ultimate desired temperature (i) is achieved, the material may be maintained at such temperature for any specified length of time (iii). In an embodiment, (iii) is from 1 minute to 4 hours, or from 30 minutes to 2 hours.

Eventually, the material is preferably cooled from the desired temperature back down to an equilibrium temperature for use in processing the material further. This cooling can occur at any rate (iv), it being understood that the cooling may be continuous or in a stepwise fashion and might be under application of an N2 atmosphere and / or under reduced pressure. The final equilibrium temperature (v), meanwhile, is preferably at or near ambient or room temperature.

### Examples

### Example 1: Production of PHBV powder

PHBV comprising about 2 wt.% valeric acid (Enmat Y1000P from Helian Polymers, NL) granulate was milled to fine powder with a cryogenic mill. By sieving the over a 100 µm sieve, powder with a particle size D50 of 46 µm was obtained. Subsequently, the powder was mixed with 0.25 wt.% carbon black in a Klarstein blender for 3 minutes at speed 12.

### Example 2: Production of PHBH powder

PHBH comprising about 6 wt.% hexanoic acid (X131A, Kaneka Belgium) granulate was first dried for 1 hour and next the temperature was heated to 110 °C and maintained at that temperature for an additional 1 hour. After this, the temperature was increased stepwise to 115 °C, 120 °C, and 125 °C, whereby the temperature at each aforementioned step was maintained for 1 hour before increasing the temperature further. After this, the temperature was raised one additional time to 130 °C, which temperature was maintained for 1 hour. After the last step, the granulate was cooled down. The granulate was milled to a fine powder with a cryogenic mill. By sieving the over a 100 µm sieve, powder with a particle size D50 of 58 µm was obtained. Subsequently, the powder was mixed with 0.25 wt.% carbon black in a Klarstein blender for 3 minutes at speed 12.

### Example 3: Production of dental aligner molds

The black powders produced in examples 1 and 2 were used to print dental molds on a Lisa Pro (Sinterit, PL) printer.

As a comparative example, a part was made via pressure molding of PHBH 151C granulate (Kaneka, Belgium) at 155 °C for 5 minutes followed by slowly cooling down. A (5 × 1 × 1 cm) was cut out of the molded part and placed in fresh plaster. After hardening of the plaster the PHBH bar was be removed and could be put back easily into the plaster mold. Subsequently. the PHBH bar was subjected to thermoforming with a CA foil. After the thermoforming the bar did not fit anymore in the plaster mold since it had started melting and had changed in geometry. In case of production of a dental aligner, such melting of the mold edges would be unacceptable since the resulting aligner would not fit the teeth.

As a comparative example, a dental aligner mold was made by FFF printing of a PLA filament. PLA filament with diameter of 1.75mm from Makerspoint was used on a RepRap X500 FFF printer equipped with a 0.2 mm nozzle diameter. The print bed was set to 40 °C and the nozzle temperature was set to 200 °C. The filament spool was clamped and transported through the printhead. The layer height was set to 0.2 mm and the layer width to 0.4 mm. The mold was printed at a print velocity of 12 mm/s. Similarly to the PHBH bar, the PLA dental aligner mold started melting and deformed when being subjected to thermoforming conditions used in the production of dental aligners.

### Example 4: Production of dental aligners by thermoforming

The dental aligner molds made in example 3 were used to produce a dental aligner by thermoforming of CA Clear aligner medium (PET-G) foils of 0.625 mm thickness (Scheu-dental, GE), using a Biostar thermoforming machine. The process guidelines as provided by the supplier were followed. The dental aligner molds were inspected by visual observation before and after the thermoforming process. Results are shown in Table 1.

**Table 1. Thermoforming results with various polymers**

| Experiment no. | Material | Melting point¹⁾ (°C) | Observations during thermoforming |
|---|---|---|---|
| 1 | PHBV Y1000P | 175 | No change in geometry |
| 2 | PHBH X131A | 145 | No change in geometry |
| A (comparative) | PHBH 151C | 130 | Started melting, change of geometry, did not fit in plaster mold anymore |
| B (comparative) | PLA | 160 | Started melting |
| C (comparative) | PA12 | 178 | Generally known to be suited for thermo forming aligner molds |

### 1) Melting point provided by supplier

It was observed that sample 1, PHBV Y1000P with a melting point of 175 °C, survived the thermoforming process without a visible change in geometry. Also sample 2, PHBH X131A having a melting point of 145 °C, survived the thermoforming process. Contrarily, PLA having a melting point of 160 °C, was not suitable as dental aligner mold, because it started to melt during the thermoforming process. PHBH 151C with a melting point of 130 °C also started to melt during the thermoforming process and is not a suitable material.

### Example 5: Dimensional accuracy of dental aligner molds

Black PHBx powders prepared according to examples 1 and 2 were used to produce dental aligner molds using different SLS printers. Print conditions are mentioned in table 2.

In experiment nos. 3a, 3b and 3c, a Lisa X printer (Sinterit, PL) was used to make dental aligner molds from PHBV powder prepared according to example 1. To study the influence of the printing position in the build, aligner molds were printed in the top, middle and bottom of the powder bed, respectively. In experiment 4, a Prodways P2000ST 3D printer equipped with a 100W CO2 laser and a counter rotating roller as recoating system was used to make dental aligner molds from PHBV powder prepared according to example 1. In experiment 5, a Lisa Pro printer (Sinterit, PL) was used to make dental aligner molds from PHBH powder prepared according to example 2. After printing, the dental aligner molds were used to produce a dental aligner by thermoforming of CA Clear aligner medium (PET-G) foils of 0.625 mm thickness (Scheu-dental, GE), using a Biostar thermoforming machine. The process guidelines as provided by the supplier were followed. Before and after the thermoforming process the dimensions of molds were determined with a scanner and compared to the STL file dimensions, and the 80% quantile (the deviated distance from the STL file covering 80% of the measured points) was calculated. Only a small deviation between before and after thermoforming was observed, showing that the dental aligner molds are stable enough to survive the thermoforming process without losing dimensional accuracy. Table 2 shows an overview of the results.

**Table 2. Overview of printed PHBx molds and dimensions before and after thermoforming**

| Experiment no. | material | Printer type | Bed temperature (°C) | Laser power | 80% Quantile before thermoforming (mm) | 80% Quantile after thermoforming (mm) | Deviation (mm) |
|---|---|---|---|---|---|---|---|
| 3a | PHBV Y1000P | Lisa X (top) | 164 | 0.8 of energy scale | 0.163 | 0.160 | 0.003 |
| 3b | PHBV Y1000P | Lisa X (middle) | 164 | 0.8 of energy scale | 0.132 | 0.131 | -0.001 |
| 3c | PHBV Y1000P | Lisa X (bottom) | 164 | 0.8 of energy scale | 0.128 | 0.128 | 0.000 |
| 4 | PHBV Y1000P | Prodways P2000ST | 150 | 30 Watt | 0.119 | 0.118 | -0.001 |
| 5 | PHBH X131A | Lisa Pro | 130 | 1.0 of energy scale | 0.224 | 0.230 | 0.006 |

## Claims

1. A positive model of one or more teeth and/or gum, suitable for producing a dental aligner, the model comprising a polyhydroxyalkanoate (PHA) having a melting peak temperature of 135 °C or higher measured according to ISO 11357-1/3, wherein the amount by weight of the PHA, relative to the total weight of the entire model, is 80-99.9 %.

2. Model according to claim 1, wherein the PHA comprises, consists essentially of, or consists of poly-3-hydroxybutyrate (P3HB), poly-4-hydroxybutyrate (P4HB), polyhydroxyvalerate (PHV), polyhydroxyhexanoate (PHH), polyhydroxyoctanoate (PHO), polyhydroxydecanoate (PHD), polyhydroxydodecanoate (PHDD), or any copolymer thereof.

3. Model according to claim 1 or 2, wherein the PHA comprises, consists essentially of, or consists of a homopolymer or copolymer of 3-hydroxy butyric acid, preferably a copolymer of 3-hydroxy butyric acid, with 3-hydroxy hexanoic acid and/or 3-hydroxy valeric acid as comonomer.

4. Model according to any one of claims 1-3, wherein the PHA has a melting peak temperature of 140 °C or higher.

5. Model according to any one of claims 1-4, further comprising one or more additives, preferably selected from flow aids, fillers, pigments, and/or stabilizers.

6. Model according to claim 5, wherein the one or more additives are present in an amount by weight, relative to the total weight of the entire model, from 0.05-20 %.

7. An additive manufacturing method for producing a model according to any one of claims 1-6, comprising sintering a powder comprising a polyhydroxyalkanoate (PHA).

8. Additive manufacturing method according to claim 7, comprising the steps of:
(a) providing a powder having a median D50 particle size value of 20-150 µm determined via laser diffraction particle size analysis in accordance with ISO 13320-1, comprising a polyhydroxyalkanoate (PHA), the powder having a crystallization onset temperature T_{c,onset} and a melting peak temperature T_{m,peak} measured according to ISO 11357-1/3,
(b) subjecting the powder to powder bed fusion, wherein the temperature of the powder bed is between the crystallization onset temperature and the melting peak temperature, thereby forming a physical model of the one or more teeth and/or gum.

9. Additive manufacturing method according to claim 7 or 8, wherein the PHA is a homopolymer or copolymer of 3-hydroxy butyric acid, preferably a copolymer of 3-hydroxy butyric acid, with 3-hydroxy hexanoic acid and/or 3-hydroxy valeric acid as comonomer.

10. Additive manufacturing method according to any one of claims 7-9, wherein the method further comprises providing a digital 3D model of one or more teeth and/or gum according to which the physical model is made, and wherein the 80% quantile of the model formed in step (b) compared to the digital 3D model is 0.5 mm or less, preferably 0.3 mm or less, more preferably 0.2 mm or less.

11. Additive manufacturing method according to any one of claims 7-10, wherein the powder composition further comprises a pigment, and wherein the amount of pigment relative to the total weight of the powder is 2.0 % or less.

12. Additive manufacturing method according to any one of claims 7-11, wherein the powder has a reflectance at a wavelength of 800 nm of 50 % or less, preferably 30 % or less, more preferably 25 % or less.

## Patentansprüche

1. Positivmodell eines oder mehrerer Zähne und/oder des Zahnfleisches, geeignet zur Herstellung eines zahnärztlichen Ausrichters, wobei das Modell ein Polyhydroxyalkanoat (PHA) mit einer Schmelzspitzentemperatur von 135 °C oder höher, gemessen nach ISO 11357-1/3, umfasst, wobei die Gewichtsmenge des PHA, bezogen auf das Gesamtgewicht des gesamten Modells, 80-99,9 % ist.

2. Modell nach Anspruch 1, wobei das PHA umfasst, im Wesentlichen besteht aus oder besteht aus Poly-3-hydroxybutyrat (P3HB), Poly-4-hydroxybutyrat (P4HB), Polyhydroxyvalerat (PHV), Polyhydroxyhexanoat (PHH), Polyhydroxyoctanoat (PHO), Polyhydroxydecanoat (PHD), Polyhydroxydodecanoat (PHDD) oder ein/einem beliebiges/nCopolymer davon.

3. Modell nach Anspruch 1 oder 2, wobei das PHA umfasst, im Wesentlichen besteht aus oder besteht aus ein/einem Homopolymer oder Copolymer von 3-Hydroxybuttersäure, vorzugsweise ein/einem Copolymer von 3-Hydroxybuttersäure, mit 3-Hydroxyhexansäure und/oder 3-Hydroxyvaleriansäure als Comonomer.

4. Modell nach einem der Ansprüche 1 bis 3, wobei das PHA eine Schmelzspitzentemperatur von 140 °C oder höher hat.

5. Modell nach einem der Ansprüche 1 bis 4, ferner umfassend einen oder mehrere Zusatzstoffe, vorzugsweise ausgewählt aus Fließhilfsmitteln, Füllstoffen, Pigmenten und/oder Stabilisatoren.

6. Modell nach Anspruch 5, wobei ein oder mehrere Zusatzstoffe in einer Gewichtsmenge, bezogen auf das Gesamtgewicht des gesamten Modells, von 0,05-20 % vorhanden sind.

7. Additives Herstellungsverfahren zum Herstellen eines Modells nach einem der Ansprüche 1 bis 6, umfassend Sintern eines Pulvers, umfassend ein Polyhydroxyalkanoat (PHA).

8. Additives Herstellungsverfahren nach Anspruch 7, umfassend die Schritte von:
(a) Bereitstellen eines Pulvers mit einem mittleren D50-Teilchengrößenwert von 20-150 µm, bestimmt mittels Laserbeugungs-Teilchengrößenanalyse in Übereinstimmung mit ISO 13320-1, umfassend ein Polyhydroxyalkanoat (PHA), wobei das Pulver eine Kristallisationsbeginn-Temperatur T_{c,onset} und eine Schmelzspitzentemperatur T_{m,peak}, gemessen in Übereinstimmung mit ISO 11357-1/3, hat,
(b) Unterziehen des Pulvers einer Pulverbettschmelze, wobei die Temperatur des Pulverbettes zwischen der Kristallisationsbeginn-Temperatur und der Schmelzspitzentemperatur ist, wodurch ein physikalisches Modell eines oder der mehrerer Zähne und/oder des Zahnfleisches gebildet wird.

9. Additives Herstellungsverfahren nach Anspruch 7 oder 8, wobei das PHA ein Homopolymer oder Copolymer von 3-Hydroxybuttersäure, vorzugsweise ein Copolymer von 3-Hydroxybuttersäure, mit 3-Hydroxyhexansäure und/oder 3-Hydroxyvaleriansäure als Comonomer ist.

10. Additives Herstellungsverfahren nach einem der Ansprüche 7 bis 9, wobei das Verfahren ferner das Bereitstellen eines digitalen 3D-Modells eines oder mehrerer Zähne und/oder des Zahnfleisches umfasst, nach dem das physikalische Modell hergestellt wird, und wobei das 80%-Quantil des in Schritt (b) gebildeten Modells verglichen mit dem digitalen 3D-Modell 0,5 mm oder weniger, vorzugsweise 0,3 mm oder weniger, stärker bevorzugt 0,2 mm oder weniger ist.

11. Additives Herstellungsverfahren nach einem der Ansprüche 7 bis 10, wobei die Pulverzusammensetzung ferner ein Pigment umfasst, und wobei die Menge des Pigments bezogen auf das Gesamtgewicht des Pulvers 2,0 % oder weniger ist.

12. Additives Herstellungsverfahren nach einem der Ansprüche 7 bis 11, wobei das Pulver einen Reflexionsgrad bei einer Wellenlänge von 800 nm von 50 % oder weniger, vorzugsweise 30 % oder weniger, stärker bevorzugt 25 % oder weniger hat.

## Revendications

1. Modèle positif d'une ou plusieurs dents et/ou d'une gencive, convenant pour la fabrication d'un aligneur dentaire, le modèle comprenant un polyhydroxyalcanoate (PHA) ayant une température de pic de fusion de 135 °C ou plus, mesurée selon la norme ISO 11357-1/3, dans lequel la quantité en poids du PHA, relativement au poids total du modèle entier, est de 80 à 99,9 %.

2. Modèle selon la revendication 1, dans lequel le PHA comprend, se compose essentiellement de, ou se compose de poly-3-hydroxybutyrate (P3HB), de poly-4-hydroxybutyrate (P4HB), de polyhydroxyvalérate (PHV), de polyhydroxyhexanoate (PHH), de polyhydroxyoctanoate (PHO), de polyhydroxydécanoate (PHD), de polyhydroxydodécanoate (PHDD), ou de n'importe quel copolymère de ceux-ci.

3. Modèle selon la revendication 1 ou 2, dans lequel le PHA comprend, se compose essentiellement de, ou se compose d'un homopolymère ou d'un copolymère d'acide 3-hydroxybutyrique, préférablement un copolymère d'acide 3-hydroxybutyrique, avec de l'acide 3-hydroxyhexanoïque et/ou de l'acide 3-hydroxyvalérique comme comonomère.

4. Modèle selon l'une quelconque des revendications 1 à 3, dans lequel le PHA a une température de pic de fusion de 140 °C ou plus.

5. Modèle selon l'une quelconque des revendications 1 à 4, comprenant en outre un ou plusieurs additifs, sélectionnés préférablement parmi des agents d'écoulement, des charges, des pigments et/ou des stabilisants.

6. Modèle selon la revendication 5, dans lequel les un ou plusieurs additifs sont présents dans une quantité en poids, relativement au poids total de l'ensemble du modèle, de 0,05 à 20 %.

7. Procédé de fabrication additive conçu pour la fabrication d'un modèle selon l'une quelconque des revendications 1 à 6, comprenant le frittage d'une poudre contenant un polyhydroxyalcanoate (PHA).

8. Procédé de fabrication additive selon la revendication 7, comprenant les étapes consistant à :
(a) fournir une poudre ayant une valeur granulométrique médiane D50 de 20 à 150 µm déterminée par analyse granulométrique par diffraction laser selon la norme ISO 13320-1, comprenant un polyhydroxyalcanoate (PHA), la poudre ayant une température de début de cristallisation T_{début,c} et une température de pic de fusion T_{pic,f} mesurée selon la norme ISO 11357-1/3,
(b) le fait de soumettre la poudre à une fusion sur lit de poudre, la température du lit de poudre étant comprise entre la température de début de cristallisation et la température de pic de fusion, en formant ainsi un modèle physique des une ou plusieurs dents et/ou de la gencive.

9. Procédé de fabrication additive selon la revendication 7 ou 8, dans lequel le PHA est un homopolymère ou un copolymère d'acide 3-hydroxybutyrique, préférablement un copolymère d'acide 3-hydroxybutyrique, avec de l'acide 3-hydroxyhexanoïque et/ou de l'acide 3-hydroxyvalérique comme comonomère.

10. Procédé de fabrication additive selon l'une quelconque des revendications 7 à 9, le procédé comprenant en outre la fourniture d'un modèle numérique 3D d'une ou plusieurs dents et/ou d'une gencive selon lequel le modèle physique est fabriqué, et le quantile à 80 % du modèle formé à l'étape (b) comparativement au modèle numérique 3D étant de 0,5 mm ou moins, préférablement de 0,3 mm ou moins, plus préférablement de 0,2 mm ou moins.

11. Procédé de fabrication additive selon l'une quelconque des revendications 7 à 10, dans lequel la composition en poudre comprend en outre un pigment, et dans lequel la quantité de pigment relativement au poids total de la poudre est de 2,0 % ou moins.

12. Procédé de fabrication additive selon l'une quelconque des revendications 7 à 11, dans lequel la poudre a une réflectance à une longueur d'onde de 800 nm de 50 % ou moins, préférablement de 30 % ou moins, plus préférablement de 25 % ou moins.
